# EUROPEAN PATENT APPLICATION

(11) **EP 4 616 798 A1**
(43) Date of publication of application: **17.09.2025**
(21) Application number: 23907402.4
(22) Date of filing: 07.11.2023
(51) Int. Cl.: A61B 5/145

(54) **METHOD FOR CORRECTING BIOMETRIC INFORMATION**

(30) Priority: 23.12.2022 KR 20220183229
(71) Applicant: i-Sens, Inc., Seoul 06646 (KR)
(72) Inventor: SEO, Jung Hee, Seoul 06646 (KR); PARK, Jeong Je, Seoul 06646 (KR); JANG, Min Ji, Seoul 06646 (KR); KANG, Yun Hee, Seoul 06646 (KR)
(74) Representative: BCKIP Part mbB
(86) International application number: PCT/KR2023/017771
(87) International publication number: WO 2024/136116

(57) **Abstract**

The present disclosure relates to a method for correcting biometric information measured in a biometric information measurement system. Further specifically, the present disclosure relates to a method for correcting the biometric information, in which an allowable range of correction information is determined based on the biometric information which is measured in a body-attachable unit at a correction time point, in which an input of inaccurate correction information may be prevented by inducing the correction information to be input within the determined allowable range, and in which the correction information may be further accurately input by reducing and variably changing the allowable range based on a degree of stabilization of the biometric information which is measured at a time of determining the allowable range of the correction information.

## Description

### Technical Field

The present disclosure relates to a method for correcting biometric information measured in a biometric information measurement system. Further specifically, the present disclosure relates to a method for correcting the biometric information, in which an allowable range of correction information is set with corrected biometric information measured at a correction time point as a reference, in which an input of inaccurate correction information may be prevented by inducing the correction information to be input within the determined allowable range, and in which the correction information may be further accurately input by variably changing the allowable range based on a degree of stabilization of the biometric information which is measured at a time of setting the allowable range of the correction information.

### Background Art

Diabetes is a chronic disease that largely occurs among contemporary people. In the Republic of Korea, two million or more people that are 5% of domestic population have diabetes.

Diabetes occurs when an absolutely large amount of a sugar component is in blood because a balance between the blood and sugar is not redressed as insulin formed in pancreas is absolutely or relatively insufficient due to various causes such as obesity, stress, a poor diet, and innate heredity.

Generally, the blood contains a specific concentration of glucose, and a tissue cell gains energy therefrom.

However, when increased more than necessary, the glucose is not appropriately stored in a muscle or a fat cell and is accumulated in the blood. Accordingly, a diabetes patient maintains blood sugar greatly higher than a normal person. As the superabundant blood sugar directly passes through tissues to be discharged in urine, a sugar component absolutely required in each of the tissues of a body becomes insufficient, which causes an abnormality in each of the tissues of the body.

Diabetes has a feature that a subjective symptom thereof is not approximately obvious at an initial stage. As diabetes progresses, unique symptoms of diabetes, such as eating much, drinking much, polyuria, weight loss, general malaise, itching, and hand and foot injuries that are not cured and last long, appear. When diabetes further progresses, complications that develop into visual disturbance, high blood pressure, kidney disease, stroke, periodontal disease, a cramp, neuralgia, gangrene, and the like appear.

In order to diagnose diabetes and manage diabetes to prevent developing the complications, systematic measurement and treatment of the blood sugar may be done simultaneously.

Since diabetes requires constant measurement of blood sugar, demand for a device associated with blood sugar measurement is constantly increased. Various researches show that occurrence of the complications of diabetes is greatly reduced when the diabetes patient strictly controls the blood sugar. Accordingly, regularly measuring the blood sugar is greatly important for the diabetes patient.

In order to control the blood sugar of the diabetes patient, such a blood gathering-type blood sugar measurement device that uses a finger prick method is mainly used. Such a blood gathering-type blood sugar measurement device helps control of the blood sugar of the diabetes patient, but accurately identifying a blood sugar value that frequently changes is difficult because the blood gathering-type blood sugar measurement device shows only a result at a time of measuring. In addition, since the blood gathering-type blood sugar measurement device always requires blood gathering in order to frequently measure the blood sugar in one day, the diabetes patient has a large burden of blood gathering.

The diabetes patient generally alternates between a hyperglycemia state and a hypoglycemia state. An emergency may occur in the hypoglycemia state. The hypoglycemia state occurs when supply of sugar does not last for a long time, and the diabetes patient may lose consciousness or may die at worst. Thus, immediately discovering the hypoglycemia state is greatly important for the diabetes patient. However, a blood gathering-type blood sugar measurement device that intermittently measures the blood sugar has a clear limitation.

In order to overcome the limitation on such a blood gathering-type blood sugar measurement device, a continuous glucose monitoring system (CGMS) that is inserted into a human body to measure the blood sugar at intervals of several minutes has been developed. Control for the diabetes patient and coping with the emergency may be facilitated with the continuous glucose monitoring system.

The continuous glucose monitoring system includes and is formed of a body-attachable unit that is attached to a body region of a user to measure blood sugar, a communication terminal that outputs a received blood sugar value, and the like. The body-attachable unit measure the blood sugar of the user, for example, for fifteen days in a state of being inserted into a body and generates blood sugar information. The body-attachable unit periodically generates the blood sugar information. A blood sugar management application may be installed in the communication terminal, and the communication terminal may periodically receive the blood sugar information and output the received blood sugar information so that the user may identify the received blood sugar information.

In order to provide accurate blood sugar information to the user, the blood sugar information received from the body-attachable unit may be initially corrected and then corrected at intervals of a correction period in a period of time of using the body-attachable unit. In initial correction, a reference blood sugar value measured through a separate blood sugar measurement device is used as correction information, so that a correction factor is calculated. A blood sugar value measured by the body-attachable unit is corrected by using the correction factor. Afterwards, a new correction factor is calculated with the reference blood sugar value measured through the blood sugar measurement device at the intervals of the correction period in the period of time of using the body-attachable unit, and the blood sugar value measured by the body-attachable unit is corrected with the new correction factor.

When the reference blood sugar value is measured by using the separate blood sugar measurement device for correction, and when the reference blood sugar value is not accurately measured because sugar is put on a finger of the user or when the user inputs a wrong reference blood sugar value by mistake even if the reference blood sugar value is accurately measured, the correction factor is not accurately calculated. Accordingly, the blood sugar value measured by the body-attachable unit is continuously inaccurately measured.

### Detailed Description of the Invention

### Technical Goals

The present disclosure is to solve the above-described difficulty of a conventional method for correcting biometric information. An aspect provides a method for correcting the biometric information, in which an allowable range of correction information is set with corrected biometric information corresponding to a correction period as a reference and in which an input of the correction information is induced within the set allowable range.

Another aspect also provides a method for correcting the biometric information, in which the allowable range is variably set based on a degree of stabilization of the biometric information which is measured at a time of setting the allowable range of the correction information.

Still another aspect also provide a method for correcting the biometric information, in which inconvenience to a user due to unrequired correction may be reduced by changing a correction period or reducing the number of times of correction after the measured biometric information is sufficiently stabilized.

### Technical solutions

According to an aspect, there is provided a method for setting an allowable range of correction information, the method including receiving at least one or more pieces of biometric information from a biometric information measurement device, generating corrected biometric information by using the received biometric information and a correction factor, extracting corrected biometric information corresponding to a correction time point from the generated corrected biometric information, and setting an allowable range of correction information that is input at the correction time point with the corrected biometric information corresponding to the correction time point as a reference.

The allowable range may be variably set by an adjustment parameter.

The adjustment parameter may be an elapsed time from a time point at which the biometric information measurement device is used, and the allowable range may variably set depending on the elapsed time.

The allowable range may be variably set to be decreased depending on the elapsed time.

The adjustment parameter may be a difference value between the corrected biometric information and the correction information, and the allowable range may be variably set based on the difference value.

The adjustment parameter may be an average value of difference values between the corrected biometric information and the correction information in a set period of time or an average value of difference values, as many as a set number of times, between the corrected biometric information and the correction information, and the allowable range may be variably set based on the average value of the difference values.

The allowable range may be variably set in proportion to the difference value or the average value.

According to another aspect, there is also provided a method for correcting biometric information measured through a biometric information measurement device, the method including setting an allowable range of correction information used to correct the biometric information, when the correction information is input at a correction time point, determining whether the correction information is valid by determining whether the input correction information is within the allowable range, and calculating a correction factor for correcting the biometric information based on the correction information depending on whether the correction information is valid.

The setting of the allowable range may include receiving at least one or more pieces of biometric information from the biometric information measurement device, generating corrected biometric information by using the received biometric information and the correction factor, extracting corrected biometric information corresponding to the correction time point from the corrected biometric information, and setting the allowable range of the correction information which is input at the correction time point based on the corrected biometric information corresponding to the correction time point.

The allowable range may be variably set by an adjustment parameter, and the adjustment parameter may be at least one of an elapsed time from a time point at which the biometric information measurement device is used and a difference value between the correction information and corrected biometric information that is corrected from the biometric information.

The calculating of the correction factor may be calculating the correction factor in one correction mode among a first correction mode and a second correction mode depending on whether the correction information is valid.

When the correction information is valid, the first correction mode may be selected as the correction mode, and in the first correction mode, the correction factor may be calculated by using the correction information which is input at the correction time point.

When the correction information is invalid, the second correction mode may be selected as the correction mode, and in the second correction mode, the correction factor may be calculated by using the correction information which is input at the correction time point and additional correction information that is additionally input at a time point close to the correction time point.

The method may further include changing a correction period when the difference value between the corrected biometric information and the correction information is within a safe allowable range, and the correction factor which is used for correcting the biometric information may be calculated in the changed correction period.

When the difference value between the corrected biometric information and the correction information is maintained within the safe allowable range a threshold number of times of change or for a change threshold time, the correction period may be changed.

The method may further include changing a correction period when the elapsed time reaches a use threshold time, and the correction factor which is used for correcting the biometric information may be calculated in the changed correction period.

### Effects of the Invention

The present disclosure has following various effects.

First, in a method for correcting biometric information according to the present disclosure, it is possible to prevent an input of inaccurate correction information by setting an allowable range of correction information with corrected biometric information corresponding to a correction period as a reference and inducing an input of the correction information within the set allowable range.

Second, in the method for correcting the biometric information according to the present disclosure, it is possible to further accurately input the correction information by reducing and variably changing the allowable range based on a degree of stabilization of the biometric information which is measured at a time of setting the allowable range of the correction information.

Third, in the method for correcting the biometric information according to the present disclosure, it is possible to reduce inconvenience to a user due to unrequired correction by changing a correction period or reducing the number of times of correction when the measured biometric information is sufficiently stabilized.

### Brief Description of Drawings

FIG. 1 is a schematic diagram illustrating a continuous biometric value measurement system according to an example embodiment of the present disclosure.
FIG. 2 is a diagram for describing an example of input of an initial correction value or a periodic correction value.
FIG. 3 is a diagram for describing a blood sugar value correction device according to the present disclosure.
FIG. 4 is a function block diagram for describing a correction management part according to an example embodiment of the present disclosure.
FIG. 5 is a flowchart for describing a correction method for biometric information according to the present disclosure.
FIG. 6 illustrates an example of data stored in a storage.
FIG. 7 is a diagram for describing an allowable range formed with an upper threshold value and a lower threshold value with a corrected blood sugar value as a reference.
FIG. 8 is a flowchart for describing an example of calculating a correction factor in the present disclosure.
FIG. 9 is a diagram for describing an example of a user interface screen for inputting a reference blood sugar value as correction information.
FIG. 10 is a diagram for describing another example of a user interface screen for inputting a reference blood sugar value as correction information.
FIG. 11 is a flowchart for describing an example of a method of changing a correction period.
FIG. 12 is a diagram for describing an example of changing a correction period.

### Mode for Carrying Out the Invention

Technical terms used in the present disclosure are merely to describe predetermined example embodiments. It should be noted that the technical terms is not to limit the present disclosure. Also, the technical terms used in the present disclosure should be construed, unless the terms are specially defined to have other meanings, as having meanings generally understood by those skilled in the art to which the present disclosure belong and should not be construed as having excessively inclusive meanings and excessively narrow meanings. In addition, when being wrong technical terms not accurately describing the idea of the present disclosure, the technical terms used in the present disclosure is to be substituted with technical terms understandable by those skilled in the art.

Also, terms in a singular form used in the present disclosure include terms in a plural form unless an apparently and contextually conflicting description is present. In the present disclosure, terms such as "including" or "comprising" should not be construed as that various elements or various operations described in the present disclosure are necessarily included. The terms should be construed as that some of the elements or the operations may not be included or that additional elements or operations may be further included.

Furthermore, the accompanying drawings are merely to easily understand the idea of the present disclosure. It should be noted that the idea of the present disclosure is not to be construed as being limited by the accompanying drawings.

Hereinafter, a correction method for biometric information according to the present disclosure will be described in detail with reference to the accompanying drawings.

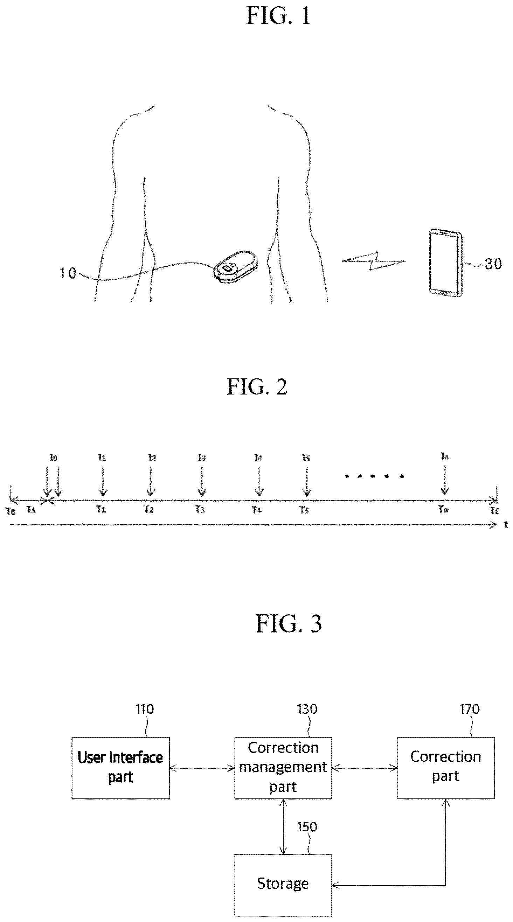

FIG. 1 is a schematic diagram illustrating a continuous biometric information measurement system according to an example embodiment of the present disclosure.

Hereinafter, a blood sugar value will be described as an example of biometric information, and a reference blood sugar value will be described as an example of reference biometric information used as correction information. However, a variety of biometric information other than the blood sugar value may be measured depending on a field to which the present disclosure is applied.

Referring to FIG. 1, a continuous biometric information measurement system 1 according to an example embodiment of the present disclosure includes a body-attachable unit 10 and a communication terminal 30.

The body-attachable unit 10 is a device attached to a body to measure the biometric information. When the body-attachable unit 10 is attached to the body, an end of a sensor of the body-attachable unit 10 is inserted into skin to measure biometric information showing the blood sugar value, namely, blood sugar information from a body fluid of a human body.

The communication terminal 30 is a terminal that may receive the blood sugar information from the body-attachable unit 10, correct the received blood sugar information with a correction factor to perform unit conversion of the blood sugar information into corrected biometric information, namely, corrected blood sugar information, and then display the corrected blood sugar information to a user. A terminal that may communicate with the body-attachable unit 10, such as a smartphone, a tablet personal computer (PC), a laptop, or the like, may be used as the communication terminal 30. Also, the communication terminal 30 is not limited thereto. Any terminal that includes a communication function and in which a program or an application may be installed may be used.

In other words, the body-attachable unit 10 generates the blood sugar information which shows the blood sugar value of the user, for example, an electric current signal, and transmits the blood sugar information to the communication terminal 30. The communication terminal 30 corrects the blood sugar information with the correction factor to perform unit conversion of the blood sugar information into a corrected blood sugar value. Depending on a field to which the present disclosure is applied, the body-attachable unit 10 may directly perform unit conversion of the blood sugar information into the blood sugar value, and the blood sugar value which is received from the body-attachable unit 10 may be corrected with the correction factor in the communication terminal 30.

Hereinafter, the blood sugar information which is measured in the body-attachable unit 10 or the blood sugar value which for which the unit conversion is performed in the body-attachable unit 10 will be referred to as a measured blood sugar value, and the blood sugar value which is obtained by correcting the blood sugar information or the unit-converted blood sugar value with the correction factor in the communication terminal 30 will be referred to as the corrected blood sugar value.

The body-attachable unit 10 transmits the blood sugar information on the measured blood sugar value to the communication terminal 30 by a request from the communication terminal 30 or periodically at each set time point. For data communication between the body-attachable unit 10 and the communication terminal 30, the body-attachable unit 10 and the communication terminal 30 may be connected in wired communication with each other by a universal serial bus (USB) cable or the like or may be connected in communication with each other with a wireless communication scheme such as infrared communication, near field communication (NFC), or Bluetooth.

When communication is connected between the body-attachable unit 10 and the communication terminal 30, after initial stabilization of the body-attachable unit 10, an initial correction factor is calculated by using, as correction information, the reference blood sugar value which is measured through an additional blood sugar measurement instrument (not illustrated), and the measured blood sugar value is corrected by using the initial correction factor. Afterwards, the communication terminal 30 outputs and provides, to the user, the corrected blood sugar value obtained by correcting, with the initial correction factor, the measured blood sugar value received from the body-attachable unit 10.

Here, with respect to the initial stabilization, the sensor is determined as being initially stabilized after a predetermined period of time, for example, after two hours elapse since being inserted into the body. Alternatively, whether the initial stabilization is performed may be determined based on the measured blood sugar value. However, the initial stabilization may be a minimum stabilization time or condition required for immediately notifying the user of the corrected blood sugar value after the sensor is inserted into the body. An additional time, for example, three to ten days may be required for actually completing stabilization of the sensor.

After the initial stabilization, in order to accurately correct the measured blood sugar value in the body-attachable unit 10, the communication terminal 30 calculates a new correction factor by using the reference blood sugar value measured through the separate blood sugar measurement instrument at intervals of a correction period in a period of time of using the body-attachable unit 10, calculates the corrected blood sugar value by correcting, by using the new correction factor, the measured blood sugar value received from the body-attachable unit, and outputs and provides the calculated corrected blood sugar value to the user.

Desirably, the correction period may be changed depending on a degree to which the stabilization has progressed. For example, correction may be performed at intervals of twelve hours after the initial stabilization, and the correction may be then performed at intervals of twenty-four hours or forty-eight hours depending on the degree to which the stabilization progressed.

FIG. 2 is a diagram for describing an example of input of initial correction information or periodic correction information. Referring to FIG. 2, a body-attachable unit is stabilized for a set stabilization time T_{S} from a time point T₀ at which the body-attachable unit and a communication terminal are connected in communication.

After initial stabilization of the body-attachable unit is completed, initial correction information I₀ is input. Here, the initial correction information I₀ may be input multiple times in order to accurately correct a blood sugar value measured in the body-attachable unit.

After stabilization of the body-attachable unit is completed, pieces of new correction information I₁, I₂, I₃, I₄, and the like are periodically input up to a time point T_{E} at which a period of time of using the body-attachable unit expires, desirably, at intervals of twelve hours or one day. A correction factor is calculated from the new correction information, and a corrected blood sugar value obtained by correcting, with the correction factor, the measured blood sugar value which is received from the body-attachable unit is provided to a user.

FIG. 3 is a diagram for describing a blood sugar value correction device according to the present disclosure.

The blood sugar value correction device according to the present disclosure may be implemented with a user terminal such as a smartphone.

Referring to FIG. 3, a correction management part 130 determines whether a correction time point arrives by monitoring a time of using a body-attachable unit and outputs a user interface screen for requesting correction information through a user interface part 110 when the correction time point arrives.

In an example embodiment of the present disclosure, when the correction time point arrives, the correction management part 130 extracts a corrected blood sugar value corresponding to the correction time point from a storage 170, calculates an allowable range of a reference blood sugar value from the extracted corrected blood sugar value, and induce an input of the reference blood sugar value within the calculated allowable range through the user interface part 110.

A correction part 150 calculates a correction factor to be used to correct a blood sugar value until a next correction time point by using the input reference blood sugar value as the correction information and, while storing the corrected blood sugar value which is corrected by using the correction factor, provides and outputs the corrected blood sugar value to a user.

Desirably, the correction management part 130 may variably set the allowable range of the reference blood sugar value. In consideration of a stabilization level of a measured blood sugar value measured in the body-attachable unit, an adjustment parameter may be determined, and the allowable range of the reference blood sugar value may be variably set by using the determined adjustment parameter. Here, the adjustment parameter may be determined based on a period of time for which the body-attachable unit is attached to a body and used, determined based on a difference value between the corrected blood sugar value and the reference blood sugar value, or determined based on a magnitude of noise of a measured signal measured in the body-attachable unit.

Desirably, when the measured blood sugar value measured in the body-attachable unit is further stabilized, the correction management part 130 may increase and change a correction period in order to reduce a pain of the user from blood collection through measurement of the reference blood sugar value due to frequent correction periods.

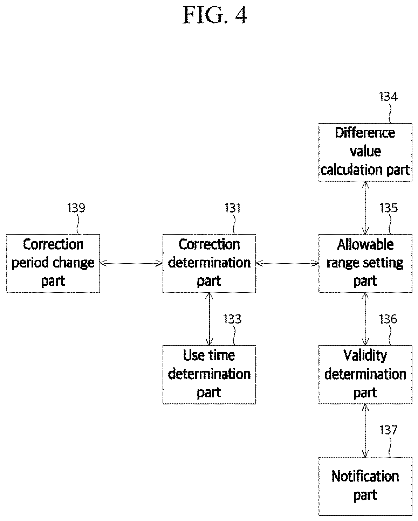

FIG. 4 is a function block diagram for describing a correction management part according to an example embodiment of the present disclosure.

Referring to FIG. 4 in detail, a correction determination part 131 determines whether a correction time point has arrived based on a correction period or based on a user instruction for requesting correction even if the correction period has not arrived.

When the correction time point has arrived, a use time determination part 133 determines a period of time of using a body-attachable unit at a current correction time point, namely, a period of time of use from a time point at which the body-attachable unit has been attached and begun to be used up to the current correction time point.

Meanwhile, a difference value calculation part 134 calculates a difference value between a corrected blood sugar value and an input reference blood sugar value at each correction time point until a previous correction time point and stores and manages the calculated difference value in a storage.

An allowable range setting part 135 sets an allowable range based on an adjustment parameter. The allowable range setting part 135 may set an allowable range of the reference blood sugar value by using the determined period of time of use as the adjustment parameter, set the allowable range by using the difference value stored in the storage as the adjustment parameter, or set the allowable range of the reference blood sugar value by using both the period of time of using and the difference value as the adjustment parameter.

When a new reference blood sugar value is input at the correction time point, a validity determination part 136 determines whether the input new reference blood sugar value is valid based on the set allowable range.

*When the new reference blood sugar value is valid, a correction factor is calculated by using the new reference blood sugar value, and a measured blood sugar value is corrected by using the calculated correction factor until a next correction time point.

However, when the new reference blood sugar value is invalid, the validity determination part 136 notifies a user that the new reference blood sugar value is invalid through a user interface part. Here, the new reference blood sugar value may be displayed in a different format, for example, by changing a color thereof.

Desirably, the validity determination part 136 determines a correction mode for calculating the correction factor based on whether the new reference blood sugar value is within the allowable range. When the new reference blood sugar value is valid, the validity determination part 136 selects, as the correction mode, a first correction mode in which the correction factor is calculated by using the reference blood sugar value which is input at the correction time point. Meanwhile, when the new reference blood sugar value is invalid, the validity determination part 136 selects, as the correction mode, a second correction mode in which the correction factor is calculated by using the an additional reference blood sugar value that is additionally input at a time point close to the correction time point or by using both the additional reference blood sugar value and the reference blood sugar value which is input at the correction time point.

When the reference blood sugar value which is out of the allowable range based on determination by the validity determination part 136 is input, a notification part 137 generates a message additionally requiring a reference blood sugar value at the time point close to the correction time point and outputs the message to the user interface part. An advice to sterilize a finger or the like before blood is extracted for measuring the reference blood sugar value may be included in the generated message. When the additional reference blood sugar value is input, the correction factor may be calculated by using the additional reference blood sugar value as correction information, or the correction factor may be calculated by using the additional reference blood sugar value and a previous reference blood sugar value together, for example, by using an average value of the additional reference blood sugar value and the previous reference blood sugar value.

When the body-attachable unit is disposed to a body and used for a predetermine period of time, a sensor of the body-attachable unit is stabilized, so that the measured blood sugar value which is measured in the body-attachable unit may be trusted. Even in this case, when blood of the user is frequently gathered so that correction thereof is performed, inconvenience may be caused to the user.

A correction period change part 139 may change the correction period based on the period of time of using the body-attachable unit, change the correction period based on the difference value between the corrected blood sugar value and the reference blood sugar value, and change the correction period in consideration of both the period of time of using and the difference value. When the correction period is changed in the correction period change part 139, the correction determination part 131 determines, with the changed correction period, a time point at which the correction is required.

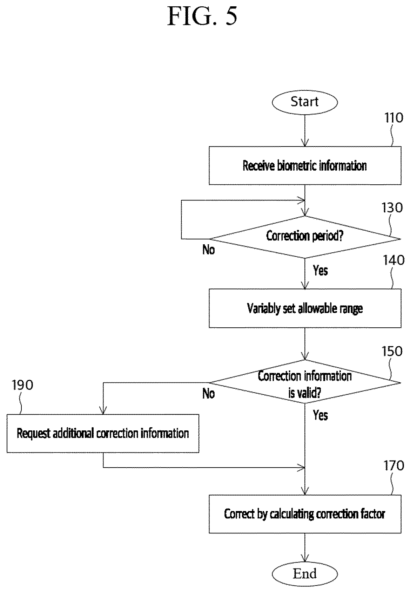

FIG. 5 is a flowchart for describing a correction method for biometric information according to the present disclosure.

Referring to FIG. 5 in detail, the biometric information, for example, a measured blood sugar value is received from a body-attachable unit in operation S110. A corrected blood sugar value is generated by correcting the received measured blood sugar value with a previously calculated correction factor and is output to a user.

In operation S130, whether a correction time point has arrived during use of the body-attachable unit is determined. Here, the correction time point may be determined with a set correction period, or a time point at which a request from the user is received regardless of the correction period may be determined to be the correction time point.

When the correction time point arrives, a correction factor is calculated by using an input reference blood sugar value as correction information. To begin with, an allowable range of the reference blood sugar value is set with a corrected blood sugar value corresponding to the correction time point as a reference in operation S140, and whether the input reference blood sugar value is valid is determined based on whether the input reference blood sugar value is within the set allowable range in operation S150.

The allowable range of the reference blood sugar value is set with the corrected blood sugar value at the correction time point as the reference. Desirably, the allowable range of the reference blood sugar value may be variably set by using an adjustment parameter with the corrected blood sugar value at the correction time point as the reference. Here, the adjustment parameter shows a stabilization level of the measured blood sugar value which is measured in a sensor of the body-attachable unit.

Further specifically, when the correction time point arrives, the allowable range of the reference blood sugar value which is input at the correction time point may be set with the corrected blood sugar value corresponding to the correction time point as the reference. Desirably, the allowable range of the reference blood sugar value which is input at the correction time point may be variably set. A period of time of using the body-attachable unit may be determined from the correction time point, and the allowable range of the reference blood sugar value may be variably set from the period of time of the using. Alternatively, the allowable range of the reference blood sugar value may be variably set from a difference value between the corrected blood sugar value and the reference blood sugar value, which is stored in a storage. Alternatively, the allowable range of the reference blood sugar value may be variably set in consideration both the period of time of the using and the difference value. Alternatively, the allowable range of the reference blood sugar value may be variably set from a noise magnitude of a measured signal measured in the sensor of the body-attachable unit. Various types of adjustment parameters may be used depending on a field to which the present disclosure is applied, which belongs to the scope of the present disclosure.

**In** operation S150, whether the reference blood sugar value is valid as the correction information is determined by determining whether the reference blood sugar value which is input as the correction information is within the set allowable range.

The allowable range may be set to various values. As an example of the allowable range, the difference value between the corrected blood sugar value and the reference blood sugar value may be used as the allowable range. When the difference value between the corrected blood sugar value and the reference blood sugar value is used as the allowable range, and when the difference value between the reference blood sugar value which is input at a current correction time point and the corrected blood sugar value which corresponds to the current correction time point is within the allowable range, the reference blood sugar value which is input as the correction information is determined as being valid.

**In** an example in which the difference value between the corrected blood sugar value and the reference blood sugar value is used as the allowable range, the allowable range may be variably set over a lapse of time from a time point at which the body-attachable unit begins to be used. Desirably, the allowable range is variably set to be decreased over a lapse of time from a time point at which the body-attachable unit is used. For example, the allowable range is set to thirty when three days elapse since the body-attachable unit is used, the allowable range is set to twenty when five days elapse, and the allowable range is set to be gradually decreased to fifteen when seven days elapse. Then, after ten days, the allowable range is set to ten. The allowable range is variably set to be decreased based on a fact that the difference value between the corrected blood sugar value and the reference blood sugar value is reduced as the body-attachable unit is stabilized over a lapse of time after use, so that the user is induced to accurately input the reference blood sugar value within the allowable range.

In an example in which the difference value between the corrected blood sugar value and the reference blood sugar value is used as the allowable range, the allowable range may be variably set based on the difference value between the corrected blood sugar value and the reference blood sugar value, which is stored in the storage.

Desirably, the allowable range may be calculated from an average value of difference values between corrected blood sugar values and reference blood sugar values in a previous set period of time before the current correction time point or may be calculated from an average value of difference values, as many as a set number of times, between the corrected blood sugar values and the reference blood sugar values.

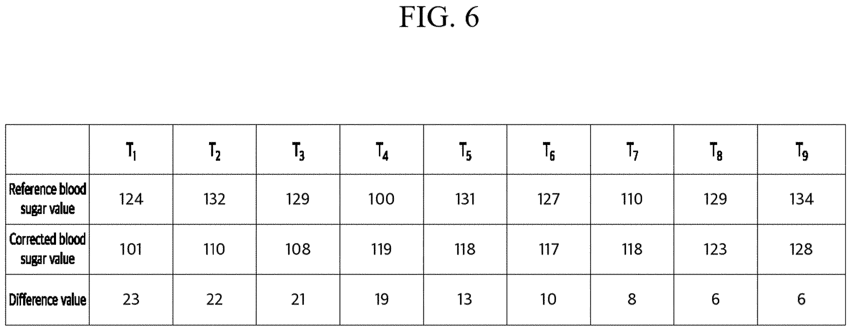

For example, as illustrated in FIG. 6, an average value of difference values (e.g., 8, 6, and 6) between corrected blood sugar values and reference blood sugar values which are calculated at previous three correction time points T₇, T₈, T₉ before a current correction time point T₁₀ may be variably set to an allowable range at the current correction time point T₁₀. Depending on a field to which the present disclosure is applied, the average value of the difference values between the corrected blood sugar values and the reference blood sugar values which are calculated at previous multiple correction time points before the current correction time point may be variably set to the allowable range at the current correction time point, or the average value of the difference values between the corrected blood sugar values and the reference blood sugar values in the previous set period of time before the current correction time point may be variably set to the allowable range at the current correction time point. This belongs to the scope of the present disclosure.

Meanwhile, as another example of the allowable range, an upper threshold value and a lower threshold value may be calculated, and the allowable range which is formed with the upper threshold value and the lower threshold value with the corrected blood sugar value as a reference may be used. When the allowable range which is formed with the upper threshold value and the lower threshold value with the corrected blood sugar value at the current correction time point as the reference is used, and when the reference blood sugar value which is input at the current correction time point is within the allowable range, the reference blood sugar value which is input as the correction information is determined to be valid.

In another example in which the allowable range which is formed with the upper threshold value and the lower threshold value with the corrected blood sugar value as the reference is used, the allowable range may be variably set over a lapse of time from the time point at which the body-attachable unit begins to be used. Desirably, the allowable range is variably set to be decreased over a lapse of time from a time point at which the body-attachable unit is used. For example, the allowable range may be set so that each of the upper threshold value and the lower threshold value with the corrected blood sugar value at the current correction time point as the reference is 15 when three days elapse since the body-attachable unit is used, the allowable range may be set so that each of the upper threshold value and the lower threshold value with the corrected blood sugar value at the current correction time point as the reference is 12 when five days elapse, and the allowable range may be set so that each of the upper threshold value and the lower threshold value with the corrected blood sugar value at the current correction time point as the reference is 7 when seven days elapse. Then, after ten days, the allowable range may be set so that each of the upper threshold value and the lower threshold value with the corrected blood sugar value at the current correction time point as the reference is 5.

In another example in which the allowable range which is formed with the upper threshold value and the lower threshold value with the corrected blood sugar value as the reference is used, the allowable range may be variably set by calculating the upper threshold value and the lower threshold value based on the difference value between the corrected blood sugar value and the reference blood sugar value, which is stored in the storage.

Desirably, the allowable range may be calculated from the average value of the difference values between the corrected blood sugar values and the reference blood sugar values in the previous set period of time before the current correction time point or may be calculated from the average value of the difference values, as many as the set number of times, between the corrected blood sugar values and the reference blood sugar values.

For example, as illustrated in FIG. 6, the average value of the difference values (e.g., 8, 6, and 6), which are calculated at the previous three correction time points T₇, T₈, and T₉ before the current correction time point T₁₀, between the corrected blood sugar values and the reference blood sugar values may be equally divided into two parts to be calculated as the upper threshold value and the lower threshold value, and the allowable range may be variably set from the upper threshold value and the lower threshold value which are calculated with a corrected blood sugar value at the current correction time point T₁₀ as a reference.

Depending on the field to which the present disclosure is applied, the average value of the difference values between the corrected blood sugar values and the reference blood sugar values which are calculated at the previous multiple correction time points before the current correction time point may be equally divided into two parts, so that the upper threshold value and the lower threshold value may be calculated. Alternatively, the average value of the difference values between the corrected blood sugar values and the reference blood sugar values in the previous set period of time before the current correction time point may be equally divided into two parts, so that the upper threshold value and the lower threshold value may be calculated.

In addition, depending on the field to which the present disclosure is applied, the average value of the difference values, which are calculated at the multiple correction time points or in the set period of time, between the corrected blood sugar values and the reference blood sugar values may be unequally distributed, so that the upper threshold value and the lower threshold value may be calculated. This belongs to the scope of the present disclosure. That is, based on a previous reference blood sugar value and a previous corrected blood sugar value, in consideration of frequency of occurrence of a difference due to the corrected blood sugar value being smaller than the reference blood sugar value and frequency of occurrence of a difference due to the corrected blood sugar value being larger than the reference blood sugar value, the average value of the difference values between the corrected blood sugar values and the reference blood sugar values which are calculated at the multiple correction time points or in the previous set period of time may be unequally divided into two parts to be calculated as the upper threshold value and the lower threshold value. For example, as illustrated in FIG. 6, based on the previous reference blood sugar value and the previous corrected blood sugar value, when the frequency of the occurrence of the difference due to the corrected blood sugar value being smaller than the reference blood sugar value is larger, the average value of the difference values between the corrected blood sugar values and the reference blood sugar values which are calculated at the multiple correction time points or in the set period of time may be calculated so that the upper threshold value is relatively larger than the lower threshold value.

Referring back to FIG. 5, in operation S170, a correction mode is selected depending on whether the reference blood sugar value which is input at the current correction time point is valid, the correction factor is calculated according to the selected correction mode, and the measured blood sugar value which is received from the body-attachable unit after the current correction time point by using the calculated correction factor is corrected.

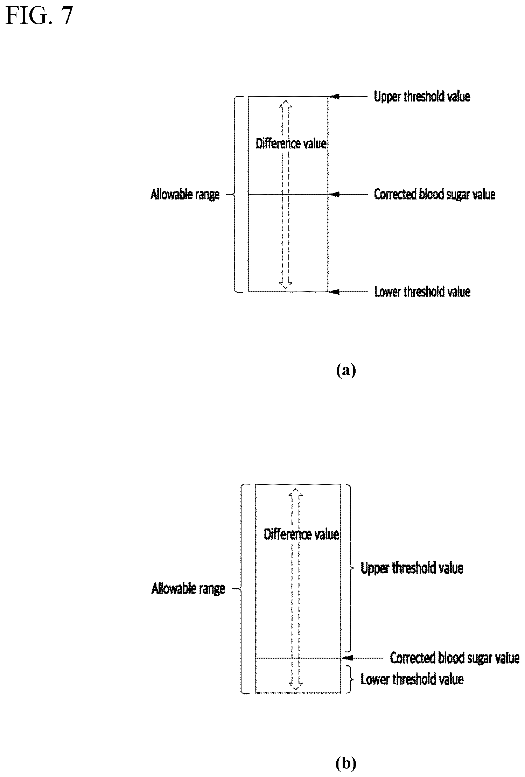

FIG. 7 is a diagram for describing an allowable range formed with an upper threshold value and a lower threshold value with a corrected blood sugar value as a reference.

As illustrated in (a) of FIG. 7, an average value of difference values between corrected blood sugar values and reference blood sugar values calculated at multiple correction time points or in a previous set time may be equally divided into two parts with a corrected blood sugar value at a current correction time point as a reference to be calculated as an upper threshold value and a lower threshold value, so that an allowable range may be set.

As illustrated in (b) of FIG. 7, the average value of the difference values between the corrected blood sugar values and the reference blood sugar values calculated at the multiple correction time points or in the previous set time before the current correction time point may be unequally divided into two parts to be calculated as the upper threshold value and the lower threshold value. Based on a reference blood sugar value and a corrected blood sugar value at the multiple correction time points or in the previous set time before the current correction time point, the average value may be unequally distributed in a ratio between frequency of occurrence of a difference due to the corrected blood sugar value being smaller than the reference blood sugar value and frequency of occurrence of a difference due to the corrected blood sugar value being larger than the reference blood sugar value, so that the upper threshold value and the lower threshold value may be calculated.

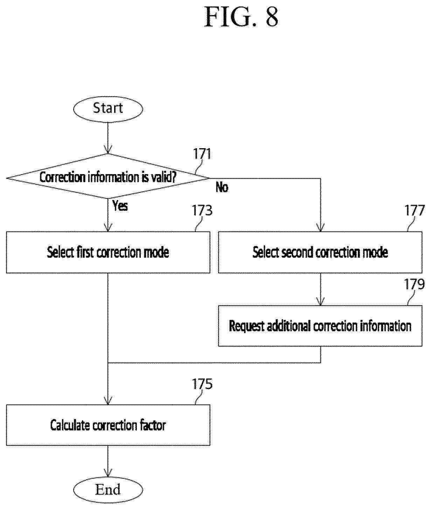

FIG. 8 is a flowchart for describing an example of calculating a correction factor in the present disclosure.

Referring to FIG. 8 in detail, in operation S171, whether a reference blood sugar value is valid as correction information is determined based on whether the reference blood sugar value which is input at a correction time point is within a set allowable range.

When the reference blood sugar value which is input at the correction time point is within the allowable range and determined as being valid, a first correction mode is selected as a correction mode in operation S173. When the first correction mode is selected as the correction mode, the correction factor is calculated by using the reference blood sugar value which is input at the correction time point in operation S175.

However, when the reference blood sugar value which is input at the correction time point is out of the allowable range and determined as being invalid, a second correction mode is selected as the correction mode in operation S177. When the second correction mode is selected as the correction mode, an additional reference blood sugar value measured at a time point close to the correction time point is requested in operation S179. A message for requesting the additional reference blood sugar value may be generated and output to a user interface, and when the additional reference blood sugar value is input, the correction factor is calculated by using both the reference blood sugar value which is input at the correction time point and the additional reference blood sugar value in operations 175.

When the additional reference blood sugar value is input in the second correction mode, the correction factor may be calculated by using the additional reference blood sugar value alone, or the correction factor may be calculated by using an average value of the reference blood sugar value at the correction time point and the additional reference blood sugar value.

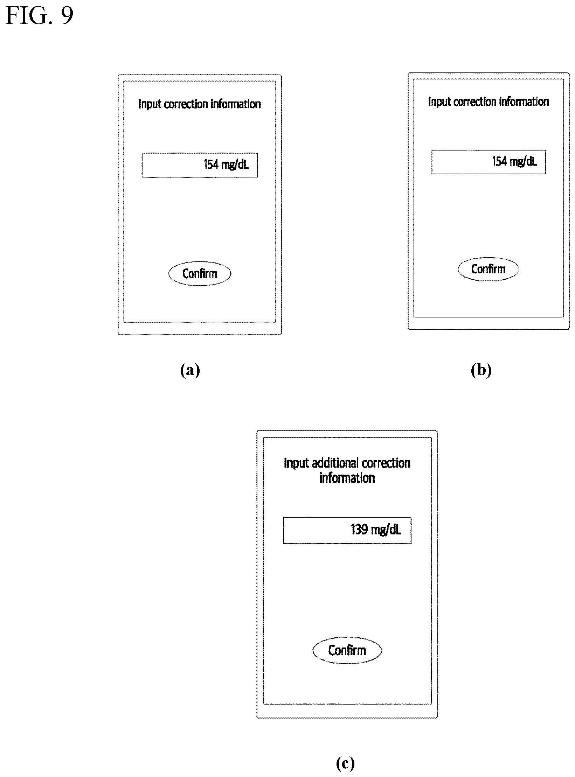

FIG. 9 is a diagram for describing an example of a user interface screen for inputting a reference blood sugar value as correction information.

As illustrated in (a) of FIG. 9, when the user interface screen for inputting the correction information is activated, the reference blood sugar value which is measured through a separate blood sugar measurement device at a correction time point is input as the correction information.

As illustrated in (b) of FIG. 9, when the input reference blood sugar value is out of an allowable range, the input reference blood sugar value is displayed in a different format, for example, in a manner in which the reference blood sugar value is changed to another color or in which a color of an input window to which the reference blood sugar value is input is changed or the like.

As illustrated in (c) of FIG. 9, when the reference blood sugar value is out of the allowable range, a user interface screen for inputting additional correction information measured through the separate blood sugar measurement device at a time point close to the correction time point is activated, and a user inputs the additional correction information to the activated user interface screen.

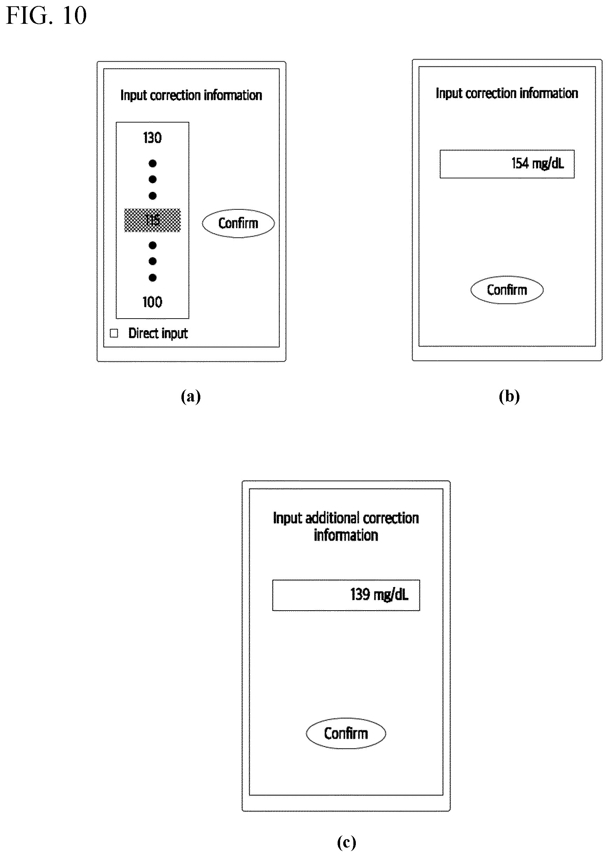

FIG. 10 is a diagram for describing another example of a user interface screen for inputting a reference blood sugar value as correction information.

As illustrated in (a) of FIG. 10, when the user interface screen for inputting the correction information is activated, a user inputs the reference blood sugar value, which is measured with a separate blood sugar measurement device at a correction time point, as the correction information in the activated user interface screen. Here, the reference blood sugar value may be input in a scrolling manner in the activated user interface screen.

As illustrated in (b) of FIG. 10, when the reference blood sugar value is out of an allowable range, or when the reference blood sugar value which is directly input by the user is out of the allowable range, the input reference blood sugar value is displayed in a different format, for example, in a manner in which the reference blood sugar value is changed to another color or in which a color of an input window to which the reference blood sugar value is input is changed or the like.

As illustrated in (c) of FIG. 10, when the reference blood sugar value is out of the allowable range, a user interface screen for inputting additional correction information is activated, and the user inputs the additional correction information to the activated user interface screen.

Depending on a field to which the present disclosure is applied, in (a) of FIG. 10, the user interface screen may be scrolled so that the reference blood sugar value is selected and input exclusively in the allowable range. In this case, the user is to directly input the reference blood sugar value in order to input the reference blood sugar value within the allowable range.

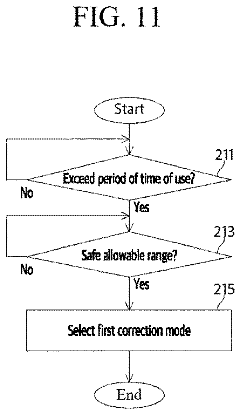

FIG. 11 is a flowchart for describing an example of a method of changing a correction period.

Referring to FIG. 11 in detail, in operation S211, whether an entire time of use exceeds a threshold time of use is determined by monitoring the entire time of use after a body-attachable unit is attached to a body and used.

When the entire time of use exceeds the threshold time of use, whether a difference value between a reference blood sugar value and a corrected blood sugar value, which is measured at a correction time point, is within a safe allowable range is determined in operation S213. Here, the safe allowable range may be set to be equal to or relatively narrower than an allowable range.

When the entire time of use of the body-attachable unit exceeds the threshold time of use, and when a difference value between a reference blood sugar value and a corrected blood sugar value, which is measured at a correction time point, is within the safe allowable range at the same time, the correction period is changed in operation S215. Desirably, the correction period is changed to be increased further than a previous correction period, so that a pain of a user from blood collection to calculate a correction factor may be reduced.

Here, when the entire time of use of the body-attachable unit exceeds the threshold time of use, a blood sugar value measured in the body-attachable unit may be determined as being stabilized, and when the difference value between the reference blood sugar value and the corrected blood sugar value, which is measured at the correction time point, is within the safe allowable range at the same time, whether the blood sugar value measured in the body-attachable unit is stabilized may be determined as being reliable.

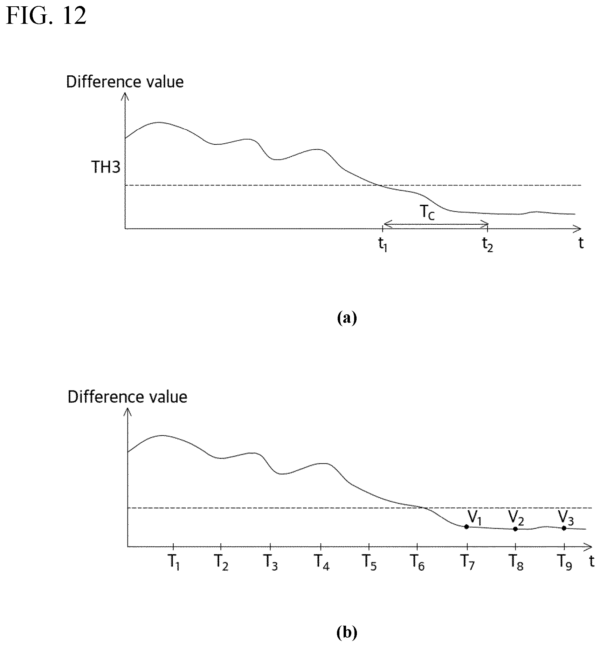

Desirably, as illustrated in (a) of FIG. 12, when the difference value between the reference blood sugar value and the corrected blood sugar value at the correction time point is maintained within a safe allowable range TH3 for a change threshold time T_{C}, the correction period is allowed to be changed.

Desirably, as illustrated in (b) of FIG. 12, when the difference value between the reference blood sugar value and the corrected blood sugar value at the correction time point is maintained a threshold number of times of change within the safe allowable range TH3, the correction period is allowed to be changed (for example, difference values V₁, V₂, and V₃ at three correction time points T7, T8, and T9 are within the safe allowable range).

Depending on a field to which the present disclosure is applied, when the entire time of use of the body-attachable unit exceeds the threshold time of use, the correction period may be changed, or when the difference value between the reference blood sugar value and the corrected blood sugar value, which is measured at the correction time point, is within the safe allowable range, the correction period is allowed to be changed. This belongs to the scope of the present disclosure.

Meanwhile, the above-described example embodiments may be prepared with a computer-executable program and implemented in a general-purpose digital computer for operating the program with a computer-readable recording medium.

The computer-readable recording medium includes a magnetic storage medium (e.g., a read-only memory, a floppy disk, a hard disk, or the like), an optical reading medium (e.g., a compact disc (CD)-ROM, a digital versatile disc (DVD), or the like), and a storage medium such as a carrier wave (e.g., transmission through the Internet).

The present disclosure has been described with reference to example embodiments illustrated in the accompanying drawings, but it is merely an example. Those skilled in the art may understand that other various modifications and equivalents are possible therefrom. Thus, the true technical scope of the present disclosure should be determined by the technical idea of the accompanying claims.

## Claims

1. A method for setting an allowable range of correction information, the method comprising:
receiving at least one or more pieces of biometric information from a biometric information measurement device;
generating corrected biometric information by using the received biometric information and a correction factor;
extracting corrected biometric information corresponding to a correction time point from the generated corrected biometric information; and
setting an allowable range of correction information that is input at the correction time point with the corrected biometric information corresponding to the correction time point as a reference.

2. The method of claim 1, wherein the allowable range is variably set by an adjustment parameter.

3. The method of claim 2, wherein the adjustment parameter is an elapsed
time from a time point at which the biometric information measurement device is used, and
the allowable range is variably set depending on the elapsed time.

4. The method of claim 3, wherein the allowable range is variably set to be decreased depending on the elapsed time.

5. The method of claim 4, wherein the adjustment parameter is a difference
value between the corrected biometric information and the correction information, and
the allowable range is variably set based on the difference value.

6. The method of claim 5, wherein the adjustment parameter is an average
value of difference values between the corrected biometric information and the correction information in a set period of time or an average value of difference values, as many as a set number of times, between the corrected biometric information and the correction information, and
the allowable range is variably set based on the average value of the difference values.

7. The method of claim 6, wherein the allowable range is variably set in proportion to the difference value or the average value.

8. A method for correcting biometric information measured through a biometric information measurement device, the method comprising:
setting an allowable range of correction information used to correct the biometric information;
when the correction information is input at a correction time point, determining whether the correction information is valid by determining whether the input correction information is within the allowable range; and
calculating a correction factor for correcting the biometric information based on the correction information depending on whether the correction information is valid.

9. The method of claim 8, wherein the setting of the allowable range comprises:
receiving at least one or more pieces of biometric information from the biometric information measurement device;
generating corrected biometric information by using the received biometric information and the correction factor;
extracting corrected biometric information corresponding to the correction time point from the corrected biometric information; and
setting the allowable range of the correction information which is input at the correction time point based on the corrected biometric information corresponding to the correction time point.

10. The method of claim 8, wherein the allowable range is variably set by an
adjustment parameter, and
the adjustment parameter is at least one of an elapsed time from a time point at which the biometric information measurement device is used and a difference value between the correction information and corrected biometric information that is corrected from the biometric information.

11. The method of claim 8, wherein the calculating of the correction factor is calculating the correction factor in one correction mode among a first correction mode and a second correction mode depending on whether the correction information is valid.

12. The method of claim 11, wherein when the correction information is valid,
the first correction mode is selected as the correction mode, and
in the first correction mode, the correction factor is calculated by using the correction information which is input at the correction time point.

13. The method of claim 11, wherein when the correction information is
invalid, the second correction mode is selected as the correction mode, and
in the second correction mode, the correction factor is calculated by using the correction information which is input at the correction time point and additional correction information that is additionally input at a time point close to the correction time point.

14. The method of claim 10, further comprising changing a correction period when the difference value between the corrected biometric information and the correction information is within a safe allowable range, wherein the correction factor which is used for correcting the biometric information is calculated in the changed correction period.

15. The method of claim 14, wherein when the difference value between the corrected biometric information and the correction information is maintained within the safe allowable range a threshold number of times of change or for a change threshold time, the correction period is changed.

16. The method of claim 10, further comprising
changing a correction period when the elapsed time reaches a use threshold time,
wherein the correction factor which is used for correcting the biometric information is calculated in the changed correction period.
